# EUROPÄISCHE PATENTSCHRIFT

(11) **EP 2 318 068 B1**
(45) Veröffentlichungstag und Bekanntmachung des Hinweises auf die Patenterteilung: **29.10.2014**
(21) Anmeldenummer: 09777950.8
(22) Anmeldetag: 18.08.2009
(51) Int. Cl.: A61M 1/16, G01G 21/22

(54) **WÄGEVORRICHTUNG EINES MEDIZINISCHEN GERÄTES, AUFNAHME HIERZU SOWIE MEDIZINISCHES GERÄT MIT EINER WÄGEVORRICHTUNG**
WEIGHING DEVICE OF A MEDICAL APPLIANCE, RECEPTACLE FOR SAME, AND MEDICAL APPLIANCE HAVING A WEIGHING DEVICE
DISPOSITIF DE PESÉE POUR UN APPAREIL MÉDICAL, RÉCEPTEUR CORRESPONDANT, ET APPAREIL MÉDICAL PRÉSENTANT UN DISPOSITIF DE PESÉE

(30) Priorität: 27.08.2008 DE 102008039916
(43) Veröffentlichungstag der Anmeldung: 11.05.2011
(73) Patentinhaber: Fresenius Medical Care Deutschland GmbH, 61352 Bad Homburg (DE)
(72) Erfinder: DITZ, Alfred, 90766 Fürth (DE); ÖSTERREICH, Stefan, 61267 Neu-Anspach (DE)
(74) Vertreter: Laufhütte, Dieter
(86) Internationale Anmeldenummer: PCT/EP2009/005984
(87) Internationale Veröffentlichungsnummer: WO 2010/022877

(56) Entgegenhaltungen:
- EP-A1- 0 610 778
- WO-A1-02/45777
- US-A- 5 147 330
- US-A1- 2006 052 738

## Beschreibung

Die vorliegende Erfindung betrifft eine Wägevorrichtung eines medizinischen Gerätes, insbesondere eine Maschine zur extrakorporalen Blutbehandlung, wie beispielsweise einer Dialysemaschine mit einer Wägeeinrichtung für die Flüssigkeitsbilanzierung. Ferner betrifft die Erfindung eine Aufnahme für eine Wägevorrichtung, ein medizinisches Gerät mit einer Wägevorrichtung, einen Behälter geeignet zu Verwendung für eine Wägevorrichtung sowie ein System umfassend eine Wägevorrichtung.

Aus dem Stand der Technik sind bereits Maschinen zur extrakorporalen Blutbehandlung, insbesondere Dialysemaschinen ohne integrierte Wasseraufbereitung bzw. "Wasserteil" bekannt, die statt dessen mit Beuteln für Dialysat, Substituat und/oder Filtrat arbeiten. Insbesondere ist der Einsatz derartiger Maschinen in der Akutdialyse, also für die Behandlung von Notfallpatienten anerkannt und verbreitet. Es kann sich z. B. um Maschinen zur Hämodialyse, Hämofiltration, Hämodiafiltration und alle artverwandten extrakorporalen Blutbehandlungsverfahren handeln. Dabei weisen derartige Geräte zur korrekten Bilanzierung der Flüssigkeitsströme von Dialysat, Substituat und/oder Filtrat beispielsweise ein Wägesystem zur gravimetrischen Flüssigkeitsbilanzierung auf. Diese Wägesysteme sind dabei derart ausgebildet, dass die Beutel für Dialysat, Substituat und/oder Filtrat in die Wägeschalen des Wägesystems eingelegt werden.

Aus der WO 02/45777 A1 ist beispielsweise eine Wägevorrichtung bekannt, in der ein Rechner der Wägevorrichtung dazu ausgelegt ist, mit einem Mikrochip eines in die Wägevorrichtung eingelegten Blutbehältnisses zu kommunizieren. Hierfür ist eine Antenne vorgesehen, die an der Wägevorrichtung befestigt ist.

Bei diesen vorbekannten Wägesystemen kann es jedoch vorkommen, dass nicht korrekt eingelegte Beutel für Dialysat, Substituat und/oder Filtrat überstehen und beispielsweise in die benachbarte Wägeschale hinüberragen und/oder die Beutel in der benachbarten Wägeschale berühren. Dadurch kann es zu einer Fehlbilanzierung kommen.

Es ist daher die Aufgabe der vorliegenden Erfindung, eine Wägevorrichtung der eingangs genannten Art in vorteilhafter Weise weiterzubilden, insbesondere dahingehend, dass eine Fehlbilanzierung durch die Wägevorrichtung sicher vermieden und die Handhabung der Wägevorrichtung vereinfacht werden kann.

Die Aufgabe wird erfindungsgemäß durch den Gegenstand des Anspruchs 1 gelöst. Danach ist vorgesehen, dass eine Wägevorrichtung eines medizinischen Gerätes wenigstens zwei Aufnahmen für abzuwiegende Behälter aufweist, wobei jeweils Seitenwandung an der Frontseite der Aufnahmen vorgesehen ist, an der eine Führungsöffnung derart ausgebildet ist, dass eine Zwangsausrichtung der Behälter realisierbar ist. Besonders vorteilhaft ist dabei, dass durch die Führungsöffnung und die dadurch in der Aufnahme herbeigeführte, definierte Lage der Behälter es ermöglicht wird, die Aufnahmen möglichst klein und damit platzsparend zu gestalten. Die Reduzierung der Baugröße der Aufnahme lässt dabei einen großen Gestaltungsspielraum bei der Auswahl von Design und Fertigungsverfahren zu. Dennoch wird vorteilhafterweise durch die Zwangspositionierung die Möglichkeit einer fehlerhaften Flüssigkeitsbilanzierung z.B. bei der Dialyse oder Akutdialyse verringert bzw. sogar ganz ausgeschlossen. Ein gegenseitiges Berühren der Behälter, die beispielsweise Dialysat, Substituat und/oder Filtrat enthalten können, wird konstruktiv verhindert. Weiter ist von Vorteil, dass die Behälter eindeutig definiert einzulegen und nach dem Einlegen in ihrer Position festgelegt sind, wodurch das Einlegen insgesamt vereinfacht wird.

Ferner ist möglich, dass die Aufnahme wenigstens eine weitere Seitenwandung aufweist, die die Aufnahme seitlich begrenzt und zusammen mit der die Führungsöffnung aufweisenden Seitenwandung einen Aufnahmeraum für den wenigstens einen Behälter bildet. Von Vorteil ist dabei, dass die zusätzliche Seitenwandung oder die zusätzlichen Seitenwandungen den Behältern weiteren Halt z.B. gegen Verrutschen bieten.

Es kann vorgesehen sein, dass die Führungsöffnung im Wesentlichen S-förmig ausgebildet ist. Diese S-förmige Führungsöffnung ermöglicht es, die Führungsöffnung beispielsweise mit einer breiten Eingangsöffnung auszuführen, die sich S-förmig geführt verengend auf einen Endbereich zubewegt, in dem beispielsweise ein Teil des Behälters wie eine Führung oder auch die Zu- bzw. Abläufe in der Führungsöffnung eingefädelt werden und durch die Hinführung zum Endbereich der Führungsöffnung für eine eigene definierte Positionierung und im Zusammenhang damit zu einer Zwangspositionierung des zugehörigen Behälters führen. Ein Herausrutschen des Behälters kann dadurch verhindert werden.

Vorteilhaft ist darüber hinaus, wenn die Führungsöffnung in einen Führungsbereich mündet, der derart ausgebildet ist, dass wenigstens ein Teil des wenigstens einen in dieser Aufnahme einzulegenden Behälters derart führbar ist, so dass sich die Zwangsausrichtung des Behälters oder der Behälter einstellt. Diese Ausgestaltung ist insbesondere von Vorteil, wenn die Führungsöffnung S-förmig ausgebildet ist. So ist es möglich, beispielsweise bei der Aufrüstung einer Dialysemaschine mit einer derartigen Wägevorrichtung die Flüssigkeitsbehälter, die vorteilhafterweise Teilbereiche wie Führungsmittel und/oder Zu- und/Ableitungen aufweisen, anhand dieser Teilbereiche geführt in die Aufnahme einzuführen, wobei durch das Führen um die S-Kurve die Zielposition des Behälters in der Aufnahme erreicht wird. Der Führungsbereich ist dabei vorteilhafterweise derart bemessen und ausgeformt, dass die Teilbereiche der Behälter aller für diese Aufnahme vorgesehenen Behälter durchgeführt werden können, also die Führungsöffnung somit durchgreifen und somit gut zugänglich sind. Genausogut kann jedoch auch vorgesehen sein, dass die einzulegenden Behälter Führungsmittel aufweisen, die in die Führungsöffnung eingesetzt werden. Dadurch wird es z.B. dem Bedienpersonal erleichtert, einen Behälter in die Aufnahme einzusetzen und geführt in die Zielposition zu verbringen, da durch die Führung der Führungsmittel in der Führungsöffnung die Führungsmittel in den Führungsbereich geführt werden, wodurch sich die Zwangspositionierung des wenigstens einen Behälters einstellt.

Denkbar ist weiterhin, dass die Aufnahmen nebeneinander angeordnet sind, wobei die Führungsöffnung sich jeweils an der Frontseite befindet und der Endbereich des Führungsbereichs jeweils innenseitig bodenseitig an der Frontseite der Aufnahme angeordnet ist. Durch eine innenseitig bodenseitige Anordnung des Endbereichs des Führungsbereichs ergibt sich der Vorteil, dass bei einer entsprechenden Ausgestaltung des Behälters der Behälterüberstand nur außenseitig auftritt und mit einfachen konstruktiven Mitteln vorteilhaft eine Berührung des Behälters mit einer benachbarten Aufnahme der Wägevorrichtung oder einem darin befindlichen weiteren Behälter vermieden werden kann. Die frontseitige Anordnung der Führungsöffnung weist den Vorteil auf, dass dadurch eine ergonomische Gestaltung der Wägevorrichtung erreicht werden kann. Das Aufrüsten kann durch somit erzielbare leichtere Zugänglichkeit vereinfacht werden.

Außerdem ist möglich, dass der Führungsbereich derart am unteren Teil der Aufnahmen ausgebildet ist, dass wenigstens ein Zu- und/oder Ablauf des in der Aufnahme einzulegenden Behälters durch den Führungsbereich hindurchführbar ist, wobei der Behälter ein Beutel, insbesondere ein Flüssigkeitsbeutel ist. So ist es möglich, beispielsweise bei der Aufrüstung einer Dialysemaschine mit einer derartigen Wägevorrichtung die Flüssigkeitsbehälter, die Zu- und Ableitungen aufweisen wie beispielsweise Schlauchanschlüsse, anhand der Zuleitung geführt in die Aufnahme einzufüh ren, wobei beispielsweise durch das Führen um die S-Kurve die Zielposition des Behälters in der Aufnahme erreicht wird. Die Zu- und/oder Ableitungen aller für diese Aufnahme vorgesehenen Behälter können dabei durch den Führungsbereich der Aufnahme durchgeführt werden können, also die Führungsöffnung somit durchgreifen.

Bevorzugt wird es, wenn die Führungsöffnung an einer Oberkante der die Führungsöffnung aufweisenden Seitenwandung beginnt und die Oberkante durchbricht. Dadurch wird es möglich, einen in die Aufnahme einzulegenden Behälter einfach von oben nach unten einzulegen, wobei das Einlegen durch die Führungsöffnung geführt wird, so dass der Behälter zwangsläufig in der Zielposition zwangspositioniert wird.

Des weiteren ist denkbar, dass die Führungsöffnung der Aufnahme jeweils nach einer Frontseite des medizinischen Gerätes ausgerichtet ist. Dadurch können zeitraubende Platzwechsel des Bedienpersonals beim Aufrüsten der Maschine vermieden werden, da z.B. eine Dialysemaschine im Wesentlichen von der Frontseite her aufgerüstet werden kann. Dabei können z. B. zwei Aufnahmen nebeneinander angeordnet sein, wobei die S-förmigen Führungsöffnungen sich jeweils an der Frontseite befinden und spiegelbildlich zueinander angeordnet sind.

Von Vorteil ist es, wenn der Behälter ein Beutel, insbesondere ein Flüssigkeitsbeutel ist. Die Ausgestaltungsform weist den Vorteil auf, dass sie besonders einfach zu handhaben ist und ferner einen geringen Staubedarf hat.

Darüber hinaus ist möglich, dass die Wägevorrichtung kopfseitig am medizinischen Gerät angeordnet ist.

Es kann vorgesehen sein, dass die Aufnahme als Wägeschale aufgeführt ist.

Ferner ist denkbar, dass die Aufnahme aus Kunststoff, beispielsweise als Kunststoffspritzgussteil oder Kunststofftiefziehteil ausgeführt ist. Dadurch wird eine einfache und kostengünstige Fertigung ermöglicht. Genausogut sind jedoch auch andere Werkstoffe und andere Fertigungsverfahren denkbar, denn die Gestaltung der Aufnahme kann mit kompakten Abmessungen erfolgen, so dass mehrere Fertigungsverfahren in Betracht kommen.

Vorteilhaft ist des Weiteren, wenn der Führungsbereich mit dem in der Aufnahme eingelegten Behälter, welcher wenigstens ein Septum aufweist, derart korrespondiert, dass das Septum durch den Führungsbereich hindurch zugänglich ist.

Des weiteren betrifft die Erfindung eine Aufnahme für eine Wägevorrichtung mit den Merkmalen des Anspruchs 13. Danach ist die Aufnahme für eine Wägevorrichtung gemäß einem der Ansprüche 1 bis 12 ausgebildet, wobei eine Seitenwandung an der Frontseite der Aufnahme vorgesehen ist, an der eine Führungsöffnung zum Hindurchführen und Einlegen eines abzuwiegenden Behälters ausgebildet und derart dimensioniert ist, dass eine Zwangsausrichtung des abzuweigenden Behälters in der Aufnahmen realisierbar ist.

Ferner betrifft die Erfindung eine Aufnahme nach Anspruch 14, wobei diese einen bodenseitig angeordneten Führungsbereich aufweist, wobei der Behälter, welcher einen Zu- und/oder Ablauf aufweist, durch den Führungsbereich hindurchführbar ist.

Außerdem betrifft die Erfindung ein medizinisches Gerät mit den Merkmalen des Anspruchs 15. Danach weist ein medizinisches Gerät, insbesondere eine Maschine zur extrakorporalen Blutbehandlung, wie beispielsweise eine Dialysemaschine, wenigstens eine Wägevorrichtung nach einem der Ansprüche 1 bis 12 auf.

Darüber hinaus betrifft die vorliegende Erfindung ein System mit den Merkmalen des Anspruchs 16. Danach ist vorgesehen, dass ein System wenigstens eine Wägevorrichtung nach einem der Ansprüche 1 bis 12 umfasst, wobei in jeweils eine Aufnahme wenigstens ein Behälter eingelegt ist.

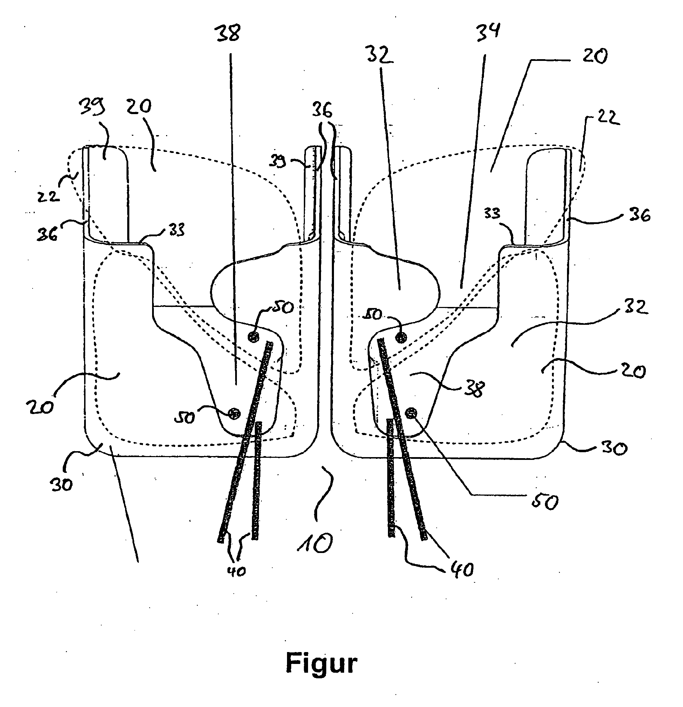

Weitere Einzelheiten und Vorteile sollen nachstehend anhand eines in der Zeichnung dargestellten Ausführungsbeispiels näher beschrieben werden.

Die einzige Figur zeigt dabei eine schematische Seitenansicht auf eine erfindungsgemäße Wägevorrichtung.

Dabei sind zwei im wesentlichen spiegelverkehrt, aber im übrigen baugleich und als Wägeschalen 30 ausgeführte Aufnahmen 30 einer Wägevorrichtung 10 gezeigt. Die Wägevorrichtung 10 ist dabei nur mit ihren Wägeschalen 30 gezeigt und ist ihrerseits kopfseitig an einer Dialysemaschine, vorzugsweise einer Akut-Dialysemaschine angeordnet. Die Wägeschalen stehen dabei lose auf jeweils einer Waage. Die Waagen sind nicht dargestellt.

In der Wägeeinrichtung 10 werden Behälter 20 eingelegt die vorzugsweise als Beutel 20 für Dialysat, Substituat und/oder Filtrat ausgeführt sind. Mittels der Wägeeinrichtung 10 werden die behandlungsbedingten Flüssigkeitsverschiebungen bilanziert, beispielsweise um den Verlauf der Behandlung zu überwachen.

Die Wägeschalen 30 weisen auf ihrer Frontseite, die nach der Frontseite der Dialysemaschine ausgerichtet ist, in der frontseitigen Seitenwandung 32 eine Führungsöffnung 34 auf, die S-förmig ausgeführt ist. Die Führungsöffnung 34 erstreckt sich von der Oberkante 33 der frontseitigen Seitenwandung 32 in den innenseitig bodenseitig befindlichen Führungsbereich 38 der S-förmigen Führungsöffnung 34. Die S-förmige Führungsöffnung 34 durchbricht dabei die Oberkante 33 der frontseitigen Seitenwandung 32.

Des weiteren weist die Wägeschale 30 weitere Seitenwandungen 36 auf, die sich auf der rechten und linken Seite der frontseitigen Seitenwandung 32 befinden. Die rückseitige Seitenwandung der Wägeschale 30 muss nicht vollständig durchgängig ausgeführt sein, sondern kann, wie in der Figur gezeigt, nur bis etwa zur halben Höhe durchgängig, jedoch im Bereich der Seitenkanten über die gesamte Höhe als versteifende Seitenkante 39 ausgeführt sein.

Die Flüssigkeitsbeutel 20, die im Zusammenhang mit der erfindungsgemäßen Wägevorrichtung 10 verwendet werden und in der Figur gestrichelt dargestellt sind, weisen einen asymmetrisch angeordneten Zu- bzw. Ablauf auf, der als Schlauchanschluss 40 oder als Zu- bzw. Ablaufschlauch 40 ausgeführt ist und sich in Bereich einer Ecke des Beutels 20 befindet. Ferner ist in räumlicher Zugehörigkeit zum Ansatz des Schlauchanschluss 40 oder des Zu- bzw. Ablaufschlauchs 40 ein Septum 50 angeordnet, durch das z.B. Medikamente und/oder Elektrolyte in das im Beutel 20 befindliche Dialysat oder Substituat zugespritzt werden können. Ferner ist es möglich, über das Septum 50 Proben aus dem Beutel 20 zu entnehmen, beispielsweise um das Filtrat analysieren zu können.

Das Einlegen der als Flüssigkeitsbeutel 20 ausgeführten Behälter 20 in die Aufnahme 30 gestaltet sich dabei folgendermaßen:

Die Beutel 20 werden von oben in die Wägeschale 30 eingebracht und mit ihren Schlauchanschlüssen 40 bzw. ihren Zu- bzw. Ablaufschläuchen 40 in die Führungsöffnung 34 eingefädelt. Durch die S-förmig ausgebildete Führungsöffnung wird der Schlauchanschluss 40 in den innenseitig bodenseitig befindlichen Führungsbereich 38 der Wägeschale 30 geführt. Da sich der Schlauchanschluss 40 in einer Ecke des Beutels 20 befindet, wird der Beutel zwangsläufig in die richtige Position innerhalb der Wägeschale 30 eingelegt. Dies gilt analog für jeden weiteren in dieser Wägeschale 30 eingelegten Beutel 20. Letztendlich können die Beutel 20 nur derart in die Wägeschale 30 eingelegt werden, dass die möglicherweise überstehenden Beutelenden 22 bzw. ein möglicher Beutelüberstand 22 von den benachbarten Wägesystemen bzw. von der benachbarten Wägeschale 30 wegzeigt bzw. wegzeigen.

Die in der Mitte der Wägeeinrichtung 10 befindlichen Seitenwandung 36 verhindern ein Überstehen der Beutel 20 über die Mitte der Wägeeinrichtung 10. Wenn überhaupt, kann im in der Figur gezeigten Ausführungsbeispiel ein Beutel 20 einen geringfügigen Beutelüberstand 22 hin zu einer Außenseite der Wägeeinrichtung 10 aufweisen, da aufgrund der innenseitig bodenseitigen Anordnung des Führungsbereichs 38 im Zusammenhang mit der innenseitigen Seitenwandung 36 die Beutel 20 nur nach außen überstehen können. Ein Überstand zur benachbarten Wägeschale wird durch die innenseitige Seitenwandung 36 verhindert, da insbesondere der obere Beutel 20, im wesentlichen schräg auf dem unteren Beutel 20 liegend, in diesem Bereich mit seinem untenliegenden Teil an der innenseitigen Seitenwandung 36 anliegt.

Der Führungsbereich 38 ist dabei vorteilhafterweise, wie auch in der Figur dargestellt, derart bemessen und gestaltet, dass die Schlauchanschlüsse 40, aber auch die Septen 50 weiterhin gut zugänglich sind. Die Kontur der Führungsöffnung 34 ermöglicht somit weiterhin, wie dies auch bei früheren Wägeeinrichtungen der Fall war, das einfache Einlegen und Entnehmen von an einer Dialysemaschine angeschlossenen Beuteln 20 und verhindert nun jedoch zusätzlich Fehlbilanzierungen und das Herausrutschen der Beutel 20 aus der Wägeschale 30.

## Patentansprüche

1. Wägevorrichtung (10) eines medizinischen Gerätes, **gekennzeichnet durch** wenigstens zwei Aufnahmen (30) für abzuwiegende Behälter (20), wobei jeweils eine Seitenwandung (32) an der Frontseite der Aufnahmen (30) vorgesehen ist, an der eine Führungsöffnung (34) derart ausgebildet ist, dass eine Zwangsausrichtung der abzuwiegenden Behälter (20) realisierbar ist.

2. Wägevorrichtung nach Anspruch 1, **dadurch gekennzeichnet, dass** die Aufnahme (30) wenigstens eine weitere Seitenwandung (36) aufweist, die die Aufnahme (30) seitlich begrenzt und zusammen mit der die Führungsöffnung (34) aufweisenden Seitenwandung (32) einen Aufnahmeraum für den wenigstens einen Behälter (20) bildet.

3. Wägevorrichtung nach Anspruch 1 oder 2, **dadurch gekennzeichnet, dass** die Führungsöffnung (34) im Wesentlichen S-förmig ausgebildet ist.

4. Wägevorrichtung nach einem der vorhergehenden Ansprüche, **dadurch gekennzeichnet, dass** die Führungsöffnung (34) in einen Führungsbereich (38) mündet, der derart ausgebildet ist, dass wenigstens ein Teil des wenigstens einen in dieser Aufnahme einzulegenden Behälters (20) derart führbar ist, so dass sich die Zwangsausrichtung des Behälters oder der Behälter (20) einstellt.

5. Wägevorrichtung nach Anspruch 4, **dadurch gekennzeichnet, dass** die Aufnahmen (30) nebeneinander angeordnet sind, wobei die Führungsöffnung (34) sich jeweils an der Frontseite (32) befindet und der Führungsbereich (38) jeweils innenseitig bodenseitig an der Frontseite (32) der Aufnahme (30) angeordnet ist.

6. Wägevorrichtung nach Anspruch 4 oder 5, **dadurch gekennzeichnet, dass** der Führungsbereich (38) derart am unteren Teil der Aufnahme (30) ausgebildet ist, dass wenigstens ein Zu- und/oder Ablauf (40) des in der Aufnahme einzulegenden Behälters (20) durch den Führungsbereich (38) hindurchführbar ist, wobei der Behälter (20) ein Beutel, insbesondere ein Flüssigkeitsbeutel ist.

7. Wägevorrichtung nach einem der vorhergehenden Ansprüche, **dadurch gekennzeichnet, dass** die Führungsöffnung (34) an einer Oberkante (33) der die Führungsöffnung (34) aufweisenden Seitenwandung (32) beginnt und die Oberkante (33) durchbricht.

8. Wägevorrichtung nach einem der vorhergehenden Ansprüche, **dadurch gekennzeichnet, dass** die Führungsöffnung (34) der Aufnahme (30) jeweils nach einer Frontseite des medizinischen Gerätes ausgerichtet ist.

9. Wägevorrichtung nach einem der vorhergehenden Ansprüche, **dadurch gekennzeichnet, dass** die Wägevorrichtung (10) kopfseitig am medizinischen Gerät angeordnet ist.

10. Wägevorrichtung nach einem der vorhergehenden Ansprüche, **dadurch gekennzeichnet, dass** die Aufnahme (30) als Wägeschale ausgeführt ist.

11. Wägevorrichtung nach einem der vorhergehenden Ansprüche, **dadurch gekennzeichnet, dass** die Aufnahme (30) aus Kunststoff und/oder als Kunststoffspritzgussteil ausgeführt ist.

12. Wägevorrichtung nach einem der Ansprüche 4 bis 11, **dadurch gekennzeichnet, dass** der Führungsbereich (38) mit dem in der Aufnahme (30) eingelegten Behälter (20), welcher wenigstens ein Septum (50) aufweist, derart korrespondiert, dass das Septum (50) durch den Führungsbereich (38) hindurch zugänglich ist.

13. Aufnahme für eine Wägevorrichtung (10) nach einem der vorhergehenden Ansprüche, **dadurch gekennzeichnet, dass** eine Seitenwandung (32) an der Frontseite der Aufnahmen (30) vorgesehen ist, an der eine Führungsöffnung (34) zum Hindurchführen und Einlegen eines abzuwiegenden Behälters (20) ausgebildet und derart dimensioniert ist, dass eine Zwangsausrichtung des abzuwiegenden Behälters (20) in der Aufnahme (30) realisierbar ist.

14. Aufnahme nach Anspruch 13, **dadurch gekennzeichnet, dass** diese einen bodenseitig angeordneten Führungsbereich (38) aufweist, wobei der Behälter (20), welcher einen Zu- und/oder Ablauf (40) aufweist, durch den Führungsbereich (38) hindurchführbar ist.

15. Medizinisches Gerät, insbesondere Dialysemaschine mit wenigstens einer Wägevorrichtung (10) nach einem der Ansprüche 1 bis 12.

16. System umfassend wenigstens eine Wägevorrichtung (10) nach einem der Ansprüche 1 bis 12, wobei in jeweils eine Aufnahme (30) wenigstens ein Behälter (20) eingelegt ist.

## Claims

1. A weighing apparatus (10) of a medical device, **characterized by** at least two receptacles (30) for containers (20) to be weighed, with a respective side wall (32) being provided at the front side of the receptacles (30at which a guide opening (34) is formed such that a forced alignment of the containers (20) to be weighed can be realized.

2. A weighing apparatus in accordance with claim 1, **characterized in that** the receptacle (30) has at least one further side wall (36) which bounds the receptacle (30) laterally and, together with the side wall (32) having the guide opening (34), forms a receiving space for the at least one container (20).

3. A weighing apparatus in accordance with claim 1 or claim 2, **characterized in that** the guide opening (34) is made substantially in S shape.

4. A weighing apparatus in accordance with one of the preceding claims, **characterized in that** the guide opening (34) opens into a guide region (38) which is configured such that at least a part of the at least one container (20) to be inserted in this receptacle can be guided such that the forced alignment of the container or of the containers (20) is adopted.

5. A weighing apparatus in accordance with claim 4, **characterized in that** the receptacles (30) are arranged next to one another, with the guide opening (34) being located in each case at the front side (32) and the guide region (38) being arranged in each case at the inner side at the base side at the front side (32) of the receptacle (30).

6. A weighing apparatus in accordance with claim 4 or claim 5, **characterized in that** the guide region (38) is configured at the lower part of the receptacle (30) such that at least one inflow and/or outflow (40) of the container (20) to be
inserted in the receptacle can be led through the guide region (38), with the container (20) being a bag, in particular a liquid bag.

7. A weighing apparatus in accordance with one of the preceding claims, **characterized in that** the guide opening (34) starts at an upper edge (33) of the side wall (32) having the guide opening (34) and breaks through the upper edge (33).

8. A weighing apparatus in accordance with one of the preceding claims, **characterized in that** the guide opening (34) of the receptacle (30) is respectively aligned in accordance with a front side of the medical device.

9. A weighing apparatus in accordance with one of the preceding claims, **characterized in that** the weighing apparatus (10) is arranged at the head side at the medical device.

10. A weighing apparatus in accordance with one of the preceding claims, **characterized in that** the receptacle (30) is made as a weighing pan.

11. A weighing apparatus in accordance with one of the preceding claims, **characterized in that** the receptacle (30) is made of plastic and/or as an injection molded plastic part.

12. A weighing apparatus in accordance with one of the claims 4 to 11, **characterized in that** the guide region (38) corresponds with the container (20) inserted in the receptacle (30) and having at least one septum (50) such that the septum (50) is accessible through the guide region (38).

13. A receptacle for a weighing apparatus (10) in accordance with one of the - preceding claims, **characterized in that** a side wall (32) is provided at the front side of the receptacle (30) at which a guide opening (34) for leading through and inserting a container (20) to be weighed is configured and dimensioned such that a forced alignment of the container (20) to be weighed can be realized in the receptacle (30).

14. A receptacle in accordance with claim 13, **characterized in that** it has a guide region (38) arranged at the base side, with the container (20), which has an inflow and/or an outflow (40), being able to be led through the guide region (38).

15. A medical device, in particular a dialysis machine, having at least one weighing apparatus (10) in accordance with one of the claims 1 to 12.

16. A system comprising at least one weighing apparatus (10) in accordance with one of the claims 1 to 12, wherein at least one container (20) is inserted into a respective one receptacle (30).

## Revendications

1. Dispositif de pesage (10) d'un appareil médical, **caractérisé par** au moins deux dispositifs de réception (30) pour les récipients (20) à peser, respectivement une paroi latérale (32) étant prévue sur la face frontale des dispositifs de réception (30), sur laquelle est réalisée une ouverture de guidage (34) de manière à pouvoir réaliser une orientation forcée des récipients (20) à peser.

2. Dispositif de pesage selon la revendication 1, **caractérisé en ce que** le dispositif de réception (30) comporte au moins une paroi latérale (36) supplémentaire, qui délimite latéralement le dispositif de réception (30) et forme, conjointement avec la paroi latérale (32) munie de l'ouverture de guidage (34), un espace de réception pour ledit au moins un récipient (20).

3. Dispositif de pesage selon la revendication 1 ou 2, **caractérisé en ce que** l'ouverture de guidage (34) est réalisée sensiblement en forme de S.

4. Dispositif de pesage selon l'une quelconque des revendications précédentes, **caractérisé en ce que** l'ouverture de guidage (34) débouche dans une zone de guidage (38) qui est réalisée de telle sorte qu'au moins une partie dudit au moins un récipient (20) à poser dans ce dispositif de réception peut être guidée de manière à établir l'orientation forcée du récipient ou des récipients (20).

5. Dispositif de pesage selon la revendication 4, **caractérisé en ce que** les dispositifs de réception (30) sont disposés l'un à côté de l'autre, l'ouverture de guidage (34) se situant respectivement sur la face frontale (32) et la zone de guidage (38) étant agencée respectivement à l'intérieur du côté du fond sur la face frontale (32) du dispositif de réception (30).

6. Dispositif de pesage selon la revendication 4 ou 5, **caractérisé en ce que** la zone de guidage (38) est réalisée au niveau de la partie inférieure du dispositif de réception (30), de telle sorte que l'entrée et/ou la sortie (40) du récipient (20) à poser dans le dispositif de réception peuvent être guidées au travers de la zone de guidage (38), ledit récipient (20) étant une poche, en particulier une poche de liquide.

7. Dispositif de pesage selon l'une quelconque des revendications précédentes, **caractérisé en ce que** l'ouverture de guidage (34) commence sur un bord supérieur (33) de la paroi latérale (32) comportant l'ouverture de guidage (34) et perce le bord supérieur (33).

8. Dispositif de pesage selon l'une quelconque des revendications précédentes, **caractérisé en ce que** l'ouverture de guidage (34) du dispositif de réception (30) est orientée dans chaque cas vers une face frontale de l'appareil médical.

9. Dispositif de pesage selon l'une quelconque des revendications précédentes, **caractérisé en ce que** ledit dispositif de pesage (10) est disposé dans la partie supérieure de l'appareil médical.

10. Dispositif de pesage selon l'une quelconque des revendications précédentes, **caractérisé en ce que** le dispositif de réception (30) est réalisé sous la forme d'une coque de pesage.

11. Dispositif de pesage selon l'une quelconque des revendications précédentes, **caractérisé en ce que** le dispositif de réception (30) est réalisé en matière plastique et/ou sous la forme d'une pièce moulée par injection de matière plastique.

12. Dispositif de pesage selon l'une quelconque des revendications 4 à 11, **caractérisé en ce que** la zone de guidage (38) correspond au récipient (20), qui est posé dans le dispositif de réception (30) et comporte au moins un septum (50), de telle sorte que ledit septum (50) est accessible en passant au travers de la zone de guidage (38).

13. Dispositif de réception pour un dispositif de pesage (10) selon l'une quelconque des revendications précédentes, **caractérisé en ce qu'**une paroi latérale (32) est prévue sur la face frontale du dispositif de réception (30), sur laquelle est réalisée une ouverture de guidage (34), qui permet de faire passer et de poser un récipient (20) à peser et qui est dimensionnée de manière à pouvoir réaliser une orientation forcée du récipient (20) à peser dans le dispositif de réception (30).

14. Dispositif de réception selon la revendication 13, **caractérisé en ce que** ledit dispositif de réception comporte une zone de guidage (38), agencée du côté du fond, le récipient (20), qui comporte une entrée et/ou sortie (40), pouvant être guidé au travers de la zone de guidage (38).

15. Appareil médical, en particulier dialyseur, comportant au moins un dispositif de pesage (10) selon l'une quelconque des revendications 1 à 12.

16. Système comportant au moins un dispositif de pesage (10) selon l'une quelconque des revendications 1 à 12, au moins un récipient (20) étant posé dans respectivement un dispositif de réception (30).
